# EUROPÄISCHE PATENTSCHRIFT

(11) **EP 1 622 587 B1**
(45) Veröffentlichungstag und Bekanntmachung des Hinweises auf die Patenterteilung: **28.04.2010**
(21) Anmeldenummer: 04730797.0
(22) Anmeldetag: 03.05.2004
(51) Int. Cl.: A61K 9/14, A61K 9/20

(54) **FESTE PHARMAZEUTISCHE ZUBEREITUNG ENTHALTEND LEVOTHYROXIN- UND/ODER LIOTHYRONINSALZE**
SOLID PHARMACEUTICAL PREPARATION CONTAINING LEVOTHYROXINE AND/OR LIOTHYRONINE SALTS
PREPARATION PHARMACEUTIQUE SOLIDE CONTENANT DES SELS DE LEVOTHYROXINE ET/OU DE LIOTHYRONINE

(30) Priorität: 02.05.2003 AT 6672003
(43) Veröffentlichungstag der Anmeldung: 08.02.2006
(73) Patentinhaber: Globopharm Pharmazeutische Produktions- und Handelsgesellschaft m.b.H., 2700 Wiener Neustadt (AT)
(72) Erfinder: BURGHART, Walter, A-1030 Wien (AT); BURGHART, Kurt, A-2700 Wiener Neustadt (AT); RANEBURGER, Johannes, A-6300 Wörgl (AT)
(74) Vertreter: Cunow, Gerda
(86) Internationale Anmeldenummer: PCT/AT2004/000150
(87) Internationale Veröffentlichungsnummer: WO 2004/096177

(56) Entgegenhaltungen:
- US-A- 5 225 204
- US-A- 5 635 209
- US-A- 5 955 105
- US-A- 5 958 979
- US-B1- 6 399 101
- SHITARA KAZUHIRO ET AL: "Solid formulations containing thyroid hormone" HCAPLUS, 2002, XP002229153

## Beschreibung

Die Erfindung bezieht sich auf eine feste pharmazeutische Zubereitung enthaltend wasserlösliche Salze von Levothyroxin und/oder Liothyronin als Wirkstoff.

Thyroidhormone der eingangs genannten Art sind in unterschiedlicher Form konfektioniert auf den Markt gebracht worden. Konventionelle Schilddrüsenhormon-Tabletten erfordern eine Reihe von Maßnahmen, um sicherzustellen, dass der Wirkstoff homogen auf alle Tabletten einer Charge verteilt und über die Zeit in gleicher Wirkstoffkonzentration vorliegt. Derartige Probleme hinsichtlich der sogenannten "content uniformity" beinhalten auch eine Reihe von Problemen mit der Stabilität des Wirkstoffes. So ist es beispielsweise bekannt, dass Levothyroxin Na-bzw. Liothyronin Nasalze dann, wenn sie als Kalziumsalze vorliegen, wasserunlöslich sind und dem Organismus nicht mehr wirksam zur Verfügung gestellt werden können. Weiters wurde eine Instabilität, und insbesondere eine Lagerinstabilität in Abhängigkeit von Feuchtigkeit, Temperatur und Licht gefunden. Es wurden daher zahllose Versuche unternommen, die Stabilität von Schilddrüsenhormon-Tabletten zu erhöhen, wobei bisher grundsätzlich der Versuch unternommen wurde, die Stabilität durch Zusatz von die Stabilität positiv beeinflussenden Hilfsstoffen zu erhöhen. In der US 5,225,204 wurde zu diesem Zweck vorgeschlagen, neben den entsprechenden Natriumsalzen wasserlösliches Polyvinylpyrrolidon einzusetzen und das Gemisch an einem Zelluloseträger zu adsorbieren, um in der Folge eine Tablette, ein Pulver oder eine Kapsel zu bilden.

In der US 5,635,209 wird neben dem Natriumsalz von Levothyroxin Na auch Natriumjodid sowie ein Sprengmittel und ein Schmiermittel eingesetzt.

In der US 5,958,979 wird als stabilisierende Komponente Natriumthiosulfat vorgeschlagen.

Aus der US 6,399,101 ergibt sich, dass die Verwendung von silizierter mikrokristalliner Zellulose Vorteile bringen soll.

Alle diese bekannten Versuche lassen zwar eine gewisse Verbesserung der Stabilität erkennen, wobei aber nach wie vor eine ausreichende Stabilität über die geforderte Lagerzeit nicht gewährleistet werden kann und insbesondere die mit Rücksicht auf die relativ geringe Dosierung von Schilddrüsenhormonen schwer einzuhaltende Homogenität nicht ohne weiteres gewährleistet wird.

Levothyroxin Na liegt in der Regel als ein bei Raumtemperatur stabiles Pentahydrat vor. Ein derartiges Pentahydrat hat bei Raumtemperatur eine gemessene Wasseraktivität von ca. 0,4 bis 0,6. Unter Wasseraktivität wird hierbei die Gleichgewichtsfeuchtigkeit verstanden, wobei 50 % relative Feuchtigkeit bei einer bestimmten Temperatur einer Wasseraktivität von 0,5 entspricht.

Die Erfindung zielt nun darauf ab, weitestgehend unabhängig von Hilfsstoffzusätzen und insbesondere ohne Zusatz von spezifischen, die Stabilität vermeintlich verbessernden Hilfsstoffen eine verbesserte Stabilität der pharmazeutischen Zubereitung, eine schnelle und einfache Herstellbarkeit derselben, eine verbesserte Homogenität der Verteilung des Wirkstoffes, sowie eine schnelle Wirkstoffdissolution (optimale Bioverfügbarkeit) zu gewährleisten. Zur Lösung dieser Aufgabe besteht die erfindungsgemäße feste pharmazeutische Zubereitung im wesentlichen darin, dass die Wasseraktivität der pharmazeutischen Zubereitung auf Werte unter 0,4, vorzugsweise 0,1 bis 0,3, gemessen bei Raumtemperatur, eingestellt ist. Überraschenderweise hat sich nämlich gezeigt, dass dann, wenn dem üblicherweise als Pentahydrat vorliegenden Wirkstoff wenigstens ein Mol Wasser entzogen wird, die Stabilität wesentlich erhöht werden kann, wobei lediglich darauf geachtet werden soll, dass in der Folge bei der Auswahl von ggf. einzusetzenden Hilfsstoffen hygroskopische Hilfsstoffe vermieden werden sollen, um eine neuerliche Aufnahme von Wasser hintanzuhalten. Dieser überraschende Effekt wird darauf zurückgeführt, dass in dem üblicherweise vorliegenden Pentahydrat ein Mol Wasser im Gegensatz zu den verbleibenden 4 Mol Wasser nicht als klassisches Hydrat vorliegt sondern in Clusterform. Eben dieses über 4 Mol Wasser hinausgehende Wasser kann leicht in die Gasphase entweichen, aber ebenso wiederum leicht in die Clusterform der Levothyroxinkristalle eingelagert werden. Dieses in Clusterform vorliegende Mol Wasser ist somit relativ leicht beweglich und nur gering an den Kristall gebunden, was sich auch durch eine entsprechend erhöhte Wasseraktivität bei Raumtemperatur zeigt. Während der Wirkstoff per se trotz dieses fünften relativ leicht beweglichen Mols Wasser hinreichend stabil ist, führt dieses volatile und leicht bewegliche, nicht im Kristall gebundene Wasser mit den üblichen für die Tablettenherstellung notwendigen Hilfsstoffen dazu, dass dieses Mol Clusterwasser zur Wechselwirkung mit, insbesondere zur Auflösung von Hilfsstoffanteilen führt und die aufgelösten Hilfsstoffanteile in Kombination mit dem freien Clusterwasser die Stabilität von Levothyroxin Na in einem nicht mehr akzeptablen Ausmaß beeinträchtigen. Liothyronin Na enthält ebenfalls bis zu 4% Wasser und zeigt das gleiche Stabilitätsverhalten wie Levothyroxin Na. Überraschenderweise hat sich nun gezeigt, dass dann, wenn dieses relativ leicht bewegliche Wasser durch Absenkung der Wasseraktivität bei Raumtemperatur auf Werte von unter 0,4, und insbesondere auf Werte von 0,1 bis 0,3 entfernt wird, auch ohne spezielle stabilisierende Hilfsstoffzusätze die entsprechende Langzeitlagerstabilität gewährleistet wird. Bei unkontrolliertem Trocknen und Absenkung der Wasseraktivitäten unter 0,1 geht Levothyroxin Na in einen amorphen Zustand über und wird dadurch wieder instabiler. Dabei ändert sich auch das Löslichkeitsverhalten im negativen Sinn zu schlechterer Dissolution. Die angegebenen optimalen Wasseraktivitäten können hierbei entweder durch entsprechend trockene Verarbeitung bei der Tablettenherstellung, durch Einsatz von entsprechend trockenen Ausgangsstoffen mit kontrollierter Wasseraktivität, wobei auch der Wirkstoff in entsprechend getrockneter Form (Wasseraktivität) zugemischt und anschließend direkt tablettiert wird, oder durch eine Trocknung der Tablettenmischung auf die erforderliche Wasseraktivität, oder durch ein gezieltes Nachtrocknen der fertigen Tabletten erzielt werden. Die Herstellung von Tabletten mit so niedrigen Wasseraktivitäten ist praktisch nicht einfach durchzuführen - großes Augenmerk muß auf das homogene Aufbringen des Wirkstoffes auf einen Träger mit möglichst großer Oberfläche gelegt werden. Durch Zugabe von entsprechenden Mengen an Tablettensprengmittel kann eine Feinadaptierung zu niedrigen Wasseraktivitäten im Bereich von 0,1 erreicht werden. Wenn eine derartige Tablette, welche beispielsweise eine Wasseraktivität von 0,1 bis 0,3 aufweisen kann, anschließend auch noch dicht verpackt wird, kann eine ausreichende Stabilität und Lagerfähigkeit gewährleistet werden. Neben der Möglichkeit einer entsprechenden dichten Verpackung kann man auch die optimale Wasseraktivität dadurch erhalten, dass größtenteils oder ausschließlich nichthygroskopische Hilfsstoffe, die für niedere Wasseraktivitäten bei Raumtemperaturen bekannt sind, wie beispielsweise Mannitol oder dgl., beim Herstellen der Tabletten verwendet werden und die Tabletten anschließend in übliche Blisterpackungen verpackt werden.

Das Problem der bei bekannten Zusammensetzungen nicht immer gewährleisteten raschen Wirkstoffdissolution und damit raschen und vollständigen Bioverfügbarkeit lässt sich in besonders einfacher Weise dadurch lösen, dass der Wirkstoff auf einen wasserlöslichen Träger homogen aufgebracht ist. Ein derartiger wasserlöslicher Träger hat in der Folge nach der Verabreichung der Tablette den Effekt, dass der Wirkstoff rasch und zuverlässig, ohne dass es erforderlich wäre, Sprengmittel einzusetzen, gelöst wird, da der schwer wasserlösliche Wirkstoff fein verteilt auf den wasserlöslichen Träger aufgebracht, gemeinsam mit dem wasserlöslichen Träger rasch in Lösung geht. Mit Vorteil wird hierbei als wasserlöslicher Träger Mannitol eingesetzt.

Um dem Problem der Aktivitätsabnahme durch Bildung wasserunlöslicher Salze zu begegnen ist es vorteilhaft, bei den zum Einsatz gelangenden Trägermaterialien, Hilfsstoffen oder Lösungsmittel, den entsprechenden Kalziumgehalt zu berücksichtigen. Mit Vorteil ist die Ausbildung hierbei so getroffen, dass der Träger mit einer dem Ca⁺⁺-Gehalt des Trägers in wesentlichen entsprechenden Menge von EDTA-Na und gegebenenfalls zusätzlichem Zusatz von Zitronensäure behandelt ist, um sicherzustellen, dass unvermeidliche Ca⁺⁺-Ionen, wie sie insbesondere in wasserlöslichen Trägern vorkommen, nicht störend wirksam werden können. Die Behandlung erfolgt aus wässeriger Lösung, wobei immer für die Entfernung des Wassers bis zur vorgegebenen Wasseraktivität zu sorgen ist. Insgesamt können die gewünschte Homogenität, die gewünschte Löslichkeit und insbesondere die Löslichkeit unter Ausbildung einer klaren Lösung die rasche Herstellbarkeit und die aufgrund der wasserfreien Arbeitsweise erhöhte Stabilität dadurch sichergestellt werden, dass die Zubereitung in Form von Tabletten vorliegt und mit nicht hygroskopischen Hilfsstoffen und/oder in einer Verpackung mit geringer oder keiner Wasserdampfdurchlässigkeit konfektioniert ist.

Das erfindungsgemäße Verfahren zur einfachen und raschen Herstellung einer pharmazeutischen Zubereitung der eingangs'genannten Art ist im wesentlichen dadurch gekennzeichnet, dass ein Träger aus Mannitol mit einer methanolischen oder alkoholischen Lösung des Wirkstoffes beladen bzw. besprüht wird, worauf das alkoholische Lösungsmittel bis zur Erzielung eines Wasserfaktors von unter 0,4, insbesondere unter 0,3 abgedampft wird und gegebenenfalls unter Zugabe von Mg-Stearat als Schmiermittel anschließend tablettiert wird. Durch die Verwendung alkoholischer Lösungsmittel und insbesondere methanolischer oder ethanolischer Lösungen der Natriumsalze wird eine entsprechend homogene Verteilung des Wirkstoffes am Träger gewährleistet. Probleme der homogenen Verteilung des Wirkstoffes in den Tabletten ("content uniformity") sind somit vermeidbar. Nach dem Abdampfen des Lösungsmittels ist eine entsprechend homogene Verteilung des wasserlöslichen Salzes am Träger sichergestellt und dadurch, dass nun die Einhaltung des geforderten Wasserfaktors überprüft wird, kann unmittelbar das stabile Produkt erzielt werden. Das Abdampfen von Restfeuchtigkeit gemeinsam mit dem Trocknungsvorgang zum Abdampfen des Lösungsmittels, nämlich Methanol und/oder Ethanol, für das wasserlösliche Salz von Levothyroxin Na bzw. Liothyronin Na führt hierbei rasch zur Einhaltung des gewünschten Wasserfaktors, wobei insbesondere bei den methanolischen oder ethanolischen Lösungen im Zuge des Verdampfens eine Art Schleppwirkung für den Abtransport des überschüssigen Wassers beobachtet werden kann, sodass eine rasche Trocknung erfolgt.

Wenn im Zuge des Tablettierungsvorganges auf weitere Hilfsstoffe, wie z.B. hygroskopische Tablettensprengmittel, verzichtet werden soll, kann so vorgegangen werden, dass als Träger direkt tablettierbares Mannitol, insbesondere Pearlitol, eingesetzt wird. Bei der Wahl von ggf. erforderlichen Tablettierungshilfsmitteln soll jedenfalls auf die Verwendung hygroskopischer Substanzen verzichtet werden, um eine Wiederanfeuchtung zu vermeiden. Mit Vorteil wird zu diesem Zweck so vorgegangen, dass als Tablettierungshilfsmittel, insbesondere Schmiermittel, hydrophobe Hilfsmittel wie z.B. Mg-Stearat eingesetzt werden.

Um, wie bereits oben erwähnt, sicherzustellen, dass in Trägermaterialien ggf. enthaltene Ca⁺⁺-Ionen nicht zur Ausbildung wasserunlöslicher Salze der Hormone führen, kann so vorgegangen werden, dass der Träger vor dem Beschichten bzw. Besprühen mit der Wirkstofflösung mit EDTA-Na, eventuell auch mit zugesetzter Zitronensäure in einer Menge versetzt wird, welche zur Komplexierung von zweiwertigen Ionen des Trägers ausreicht. Nach dem Zwischentrocknen, auch bei erhöhten Temperaturen, trägt man dann die alkoholische Wirkstofflösung auf. Direkt anschließend gibt man eine weitere Menge an wässriger Komplexierlösung wie oben beschrieben zu, um zusätzliche vorhandene Ionen aus Lösungsmittel oder aus Produktionsapparaturen zu binden. Die jeweils zur Komplexierung der Ca⁺⁺-Ionen eingesetzte EDTA-Na-Menge soll jedenfalls nicht in überschüssigen Mengen eingesetzt werden, da EDTA-Na/Zitronensäure selbst die Stabilität von Levothyroxin Na- bzw. Liothyronin Nasalzen nicht ohne weiteres erhöht.

Alternativ kann der Träger bestehend aus Mannitol, gegebenfalls mit Stärke, Guar oder anderen Granulierhilfen vermischt, mit einer methanolischen oder alkoholischen Lösung des Wirkstoffes beladen und direkt anschließend mit einer wässrigen Lösung, die gegebenenfalls EDTA-Na und/oder Zitronensäure enthält, feucht granuliert werden. Wasser wird abgetrocknet bis zur entsprechenden Wasseraktivität, gegebenenfalls mit Unterstützung durch nachfolgende Sprengmittelzugabe. Nach dem Zumischen von Tablettenschmiermittel werden Tabletten niedriger Wasseraktivität hergestellt. Die anschließende bevorzugte Verpackung sollte wasserdampfdicht sein. Die so hergestellten und bei 25°C gelagerten Tabletten sind ungewöhnlich stabil.

Die Erfindung wird nachfolgend anhand von Ausführungsbeispielen und anhand von Vergleichsversuchen näher erläutert:

### Beispiel 1:

Die wasserlöslichen Salze der Wirkstoffe wurden in einem organischen, wasserfreien Lösungsmittel wie z.B. Methanol und Ethanol gelöst. Ein wenig hygroskopischer Trägerstoff in Form von Mannitol (Pearlitol 400 DC) wurde mit der Wirkstofflösung durch Aufgießen oder Aufsprühen befeuchtet. In der Folge wurde das Lösungsmittel durch Wirbelschichttrocknen oder Evakuieren entfernt, wobei im Trocknungsvorgang ein Wasserfaktor von 0,3 eingestellt wurde. Bei der Tablettierung wurde auf Sprengmittel zur Gänze verzichtet. Als Tablettierschmiermittel wurde Mg-Stearat verwendet.

In diesem Ausführungsbeispiel wurde die nachfolgende Zusammensetzung gewählt:

| | |
|---|---|
| Pearlitol 400 DC | 2749 g |
| Methanol | 60 g |
| Levothyroxin Na | 1,632 g |
| Magnesiumstearat | 32 g |

Die so erhaltene Mischung ließ sich ohne Probleme direkt tablettieren, wobei sich hinsichtlich Aussehen, Härte, Friabilität und anderen pharmazeutischen Parametern einwandfreie Tabletten herstellen ließen. Der Tablettenzerfall fand innerhalb einer Minute statt, wobei sich bis auf das auf der Wasseroberfläche schwimmende Mg-Stearat eine klare Lösung bildete. Es ist davon auszugehen, dass eine optimale Bioverfügbarkeit der Wirkstoffe gegeben ist, da bis auf geringe Mengen unlösliches Mg-Stearat keine weiteren unlöslichen Hilfsstoffe in der Dissolutionslösung vorhanden sind.

Diese Tabletten hatten bei einer Raumtemperatur von 25°C einen Wasserfaktor von 0,2. Die Wasseraufnahme wurde in einem Versuch bei einer Lagerung von 25°C, 60% relativer Feuchte und einer Lagerdauer von 24h gegen Tabletten einer herkömmlichen Rezeptur (enthaltend z.B. Tablettensprengmittel, sowie weitere pharmazeutische Hilfsmittel), die ebenfalls auf einen Wasserfaktor von 0,2 zuvor getrocknet wurden, geprüft. Bei der erfindungsgemäßen Ausbildung wurde lediglich 0,11% Wasseraufnahme und ein Wasserfaktor von 0,3 beobachtet, während die herkömmliche Rezeptur eine Wasseraufnahme von 0,75% (fast 7x mehr) und einen Wasserfaktor von 0,5 erzielte.

### Beispiel 2:

In Beispiel 2 wurde vor dem Aufbringen der Lösung aus Methanol, Levothyroxin Na, wie sie bereits in Beispiel 1 vorgenommen wurde, die gleiche Menge an Pearlitol, nämlich 2749 g zuvor mit 60g Methanol befeuchtet und anschließend mit einer Lösung aus 40 g Wasser, 0,12 g Zitronensäure wasserfrei und 4,0 g EDTA-Dinatrium vorbehandelt. Die auf diese Weise erzielte Mischung wurde auf einen Wasserfaktor von 0,2 bis 0,25 getrocknet und anschließend tablettiert, wobei eine gute Tablettierbarkeit und ein Tablettenzerfall in einer Zeit von ungefähr 1 Minute beobachtet wurde. Die Aussagen des Beispiels 1 treffen auch hier zu. Der Wirkstoff ist, wie im Beispiel 1, homogen am Träger aufgetragen und es besteht keine Gefahr einer Entmischung ("content uniformity") . Der zusätzliche Schutz durch die aufgetragene Komplexierlösung vor zweiwertigen Ionen verhindert eine entsprechende Deaktivierung des Wirkstoffes.

### Beispiel 3:

In Beispiel 3 wurden zwei Trockungsvorgänge vorgenommen, wobei sowohl nach dem Behandeln von Pearlitol mit der zuvor beschriebenen EDTA Lösung und Methanol ein erster Trocknungsvorgang erfolgt, worauf das Auftragen des Wirkstoffes mit der zuvor beschriebenen methanolischen Lösung gleichzeitig mit einer weiteren Teilmenge der EDTA-Dinatrium Lösung erfolgte, worauf ein neuerlicher Trocknungsvorgang angeschlossen wurde.

### Beispiel 4:

An diesem Beispiel wird gezeigt, daß auch bei Herstellung mit einem konventionellen wässrigen Granulierverfahren und durch Einsatz von üblichen Tablettierhilfsstoffen wie Träger (Mannit), Granuliermittel (Guar), Tablettensprengmittel (Natriumcarboxymethylstärke), Schmiermittel (Magnesiumstearat und Talk) so wie üblichen Komplexierungsmittel (EDTA-Na, Zitronensäure) eine Tablette mit ungewöhnlicher Wirkstoffstabilität hergestellt werden kann, wenn die Wasseraktivität entsprechend den Vorgaben eingestellt ist (0,3) und für eine anschließende dichte Verpackung gesorgt wird.

In diesem Ausführungsbeispiel wurde die nachfolgende Zusammensetzung gewählt:

| | |
|---|---|
| Mannit | 11,78944 kg |
| Guar | 0,44 kg |
| Methanol | 0,3 kg |
| Levothyroxin Na | 0,00816 kg |
| Wasser | 2,5 kg |
| EDTA-Na | 0,08 kg |
| Zitronensäure | 0,0024 kg |
| Natriumcarboxymethylstärke | 1,2 kg |
| Talk | 0,32 kg |
| Magnesiumstearat | 0,16 kg |

Es wurde der mit Guar vermischte Träger mit der Wirkstofflösung beladen und anschließend mit einer wässrigen Lösung vn EDTA-Na und Zitronensäure versetzt sowie feucht granuliert, worauf auf eine Wasseraktivität unter 0,3 getrocknet wurde. Tabletten hergestellt mit einer Wasseraktivität von 0,45, in PVC verblistert bei 25°C und 60 % r.F. über 12 Monate gelagert zeigen einen Wirkstoffgehalt von nur mehr 88,6 % des deklarierten Wertes (Ausgang 100 %) und sind damit nicht mehr verkehrsfähig. Die gleichen Tabletten getrocknet auf eine Wasseraktivität von 0,3, geblistert in PVC aber in Sachets wasserdampfdicht verpackt, unter gleichen Bedingungen gelagert nach 12 Monaten, zeigen unter Berücksichtigung der normalen Analysenschwankungen den ursprünglichen Gehalt von(99,6 %). Diese Tabletten sind daher aussergewöhnlich stabil.

Für die den Beispielen 1 - 3 entsprechenden Präparate haben sich folgende Stabilitätsverbesserungen ergeben, wobei die Stabilitätsverbesserungen für verschiedene Wirkstoffkonzentrationen ermittelt wurden:

100 µg Levothyroxin Na, relative Feuchtigkeit 40 - 50 % (Wasserfaktor 0,4 bis 0,5). Der Anteil von Levothyroxin Na sank nach 3 Monaten auf 87 Gew.% ab, wobei zur Beschleunigung der Resultate Lagerbedingungen bei 40° C und 75 % relativer Feuchtigkeit gewählt wurden. Demgegenüber haben die 100 µg Levothyroxin Na enthaltenden Präparate mit einem Wasserfaktor von 0,3 nach 3 Monaten noch eine Aktivität von 92,9 % aufgewiesen. Die gleichen Lagerbedingungen und Messungen für Präparate mit 160 µg Levothyroxin Na ergaben bei einem Wasserfaktor von 0,4 bis 0,5 nach 3 Monaten 90,7 Gew.% an verbleibender Aktivität, wohingegen bei einem Wasserfaktor von 0,3 Aktivitäten von 95,2 Gew.% der ursprünglichen Menge sichergestellt werden konnten. Bei Kombinationspräparaten enthaltend Levothyroxin Na und Liothyronin Na wurden vergleichbare Verbesserungen beobachtet, wobei bei einem reinen 25 µg Liothyronin Na enthaltenden Präparat der Gehalt nach 1 Monat bei einem Wasserfaktor von 0,4 bis 0,5 bereits auf 87,2 % abgesunken war, wohingegen bei einem Wassserfaktor von 0,25 die entsprechende Analyse nach 1 Monat noch 97,7 Gew.% der eingesetzten Menge als aktiv ergeben hat.

Insgesamt hat sich eine Direkt-Tablettierung unter trockenen Bedingungen als besonders rasche und besonders einfache Verfahrensweise zur Herstellung einer entsprechend homogenen und langzeitstabilen Zusammensetzung erwiesen.

## Patentansprüche

1. Feste pharmazeutische Zubereitung enthaltend wasserlösliche Salze von Levothyroxin und/oder Liothyronin als Wirkstoff, **dadurch gekennzeichnet, dass** die Wasseraktivität der pharmazeutischen zubereitung bei Raumtemperatur- gemessen auf Werte unter 0,4 und über 0,1 eingestellt list.

2. Pharmazeutische Zubereitung nach Anspruch 1, **dadurch gekennzeichnet, dass** die Wasseraktivität der pharmazeutischen Zubereitung bei Raumtemperatur gemessen auf Werte von 0,1 bis 0,3 eingestellt ist.

3. Pharmazeutische Zubereitung nach Anspruch 1 oder 2, **dadurch gekennzeichnet, dass** der Wirkstoff aus einer methanolischen/ethanolischen Lösung homogen auf einen gegebenenfalls mit Stärke, Guar oder Granulierhilfen vermischten Träger aufgebracht ist.

4. Pharmazeutische Zubereitung nach Anspruch 1,2 oder 3, **dadurch gekennzeichnet, dass** der Wirkstoff auf einen wasserlösliche Träger aufgebracht ist.

5. Pharmazeutische Zubereitung nach einem der Ansprüche 1 bis 4 **dadurch gekennzeichnet, dass** als wasserlöslicher Träger Mannitol eingesetzt ist.

6. Pharmazeutische Zubereitung nach einem der Ansprüche 1 bis 5, **dadurch gekennzeichnet, dass** der Träger mit einer dem ca⁺⁺-Gehalt des Trägers in wesentlichen entsprechenden Menge von EDTA-Na und gegebenenfalls Zitronensäure behandelt ist.

7. Pharmazeutische Zubereitung nach einem der Ansprüche 1 bis 6, **dadurch gekennzeichnet, dass** die Zubereitung in Form von Tabletten vorliegt und mit nicht hygroskopischen Hilfsstoffen und/oder in einer Verpackung mit geringer oder keiner Wasserdampfdurchlässigkeit konfektioniert ist.

8. Verfahren zur Herstellung einer pharmazeutischen Zubereitung nach einem der Ansprüche 1 bis 7, **dadurch gekennzeichnet, dass** ein Träger aus Mannitol mit einer alkoholischen Lösung des Wirkstoffes beschichtet bzw. besprüht wird, worauf das alkoholische Lösungsmittel bis zur Erzielung eines Wasserfaktors von unter 0,4, insbesondere unter 0,3 und über 0,1 abgedampft wird und anschließend tablettiert wird.

9. Verfahren nach Anspruch 8, **dadurch gekennzeichnet, dass** vor dem Abdampfen des Lösungsmittels Wasser oder eine wässrige Lösung enthaltend EDTA-Na und/oder Zitronensäure aufgebracht wird.

10. Verfahren nach Anspruch 8 oder 9, **dadurch gekennzeichnet, dass** als Träger direkt tablettierbares, Mannitol, insbesondere Pearlitol, eingesetzt wird.

11. Verfahren nach Anspruch 8,9 oder 10, **dadurch gekennzeichnet, dass** als Tablettierungshilfsmittel, insbesondere Schmiermittel, hydrophobe Hilfsmittel wie z.B. Mg-Stearat eingesetzt werden.

12. Verfahren nach einem der Ansprüche 8 bis 11, **dadurch gekennzeichnet**, 1 dass der Träger vor dem Beschichten bzw. Besprühen mit der Wirkstofflösung mit EDTA-Na und gegebenenfalls Zitronensäure in einer Menge versetzt wird, welche zur Komplexierung von zweiwertigen Ionen des Trägers und anderen Quellen, wie Lösungsmittel, ausreicht.

13. Verfahren nach einem der Ansprüche 8 bis 12 , **dadurch gekennzeichnet, dass** der Träger vor dem Versetzen mit EDTA-Na und gegebenenfalls Zitronensäure mit Methanol angefeuchtet wird.

14. Verfahren nach einem der Anspruche 8 bis 13, **dadurch gekennzeichnet, dass** die Zusammensetzung frei von hygroskopischen Hilfsmitteln gehalten wird.

15. Verfahren nach einem der Ansprüche 8 bis 13, **dadurch gekennzeichnet, dass** die Zusammensetzung hygroskopische Hilfsmittel enthält aber in wasserdampfdichter Verpackung abgepackt ist.

## Claims

1. A solid pharmaceutical preparation containing water-soluble salts of levothyroxine and/or liothyronine as active ingredients, **characterized in that** the water activity of said pharmaceutical preparation is adjusted to values of below 0,4 and above 0,1, measured at room temperature.

2. A solid pharmaceutical preparation according to claim 1, **characterized in that** the water activity of said pharmaceutical preparation is adjusted to values ranging from 0,1 to 0,3, measured at room temperature.

3. A solid pharmaceutical preparation according to claim 1 or 2, **characterized in that** the active ingredient from a methanolic/ethanolic solution is homogenously applied on a carrier optionally mixed with starch, guar or grnulation aids.

4. A solid pharmaceutical preparation according to claim 1, 2 or 3, **characterized in that** the active ingredient is applied on a water-soluble carrier.

5. A solid pharmaceutical preparation according to any one of claims 1 to 4, **characterized in that** mannitol is used as a water-soluble carrier.

6. A solid pharmaceutical preparation according to any one of claims 1 to 5, **characterized in that** the carrier is treated with an amount of EDTA-Na substantially corresponding to the Ca⁺⁺-content of the carrier, and optionally citric acid.

7. A solid pharmaceutical preparation according to any one of claims 1 to 6, **characterized in that** the preparation is present in the form of tablets and confectioned with non-hygroscopic adjuvants and/or in a package with only little or no water-vapor permeability.

8. A method for producing a pharmaceutical preparation according to any one of claims 1 to 7, **characterized in that** a carrier of mannitol is coated or sprayed with an alcoholic solution of the active ingredient, whereupon the alcoholic solvent is evaporated until a water factor of below 0,4 and, in particular, below 0,3 and above 0,1 has been reached, and tableting is subsequently effected.

9. A method according to claim 8, **characterized in that** water or an aqueous solution containing EDTA-Na and/or citric acid is applied prior to evaporating said solvent.

10. A method according to claim 8 or 9, **characterized in that** directly tabletable mannitol and, in prticular, pearlitol is used as a carrier.

11. A method according to claim 8, 9 or 10, **characterized in that** hydrophobic adjuvants such as, e.g., magnesium stearate are used as tableting aids and, in particular, lubricants.

12. A method according to any one of claims 8 to 11, **characterized in that** the carrier, prior to being coated or sprayed with the active ingredient solution is supplemented with EDTA-Na and optionally citric acid, in an amount sufficient for complexing bivalent ions of the carrier and other sources such as solvents.

13. A method according to any one of claims 8 to 12, **characterized in that** the carrier is wetted with mannitol prior to being supplemented with EDTA-Na and optionally citric acid.

14. A method according to any one of claims 8 to 13, **characterized in that** the composition is kept free of hygroscopic adjuvants.

15. A method according to any one of claims 8 to 13, **characterized in that** the composition contains hygroscopic adjuvants, yet is packed in a water-vapor-tight package.

## Revendications

1. Préparation pharmaceutique solide contenant des sels hydrosolubles de lévothyroxine et/ou de liothyronine en tant que principe actif, **caractérisée en ce que** l'activité de l'eau de la préparation pharmaceutique mesurée à la température ambiante est établie à des valeurs inférieures à 0,4 et supérieures à 0, 1.

2. Préparation pharmaceutique selon la revendication 1, **caractérisée en ce que** l'activité de l'eau de la préparation pharmaceutique mesurée à la température ambiante est établie à des valeurs allant de 0,1 à 0,3.

3. Préparation pharmaceutique selon la revendication 1 ou 2, **caractérisée en ce que** le principe actif provenant d'une solution méthanolique/éthanolique est appliqué de manière homogène sur un excipient mélangé le cas échéant à de l'amidon, de la gomme de guar ou des auxiliaires sous forme de granulés.

4. Préparation pharmaceutique selon la revendication 1, 2 ou 3, **caractérisée en ce que** le principe actif est appliqué sur un excipient hydrosoluble.

5. Préparation pharmaceutique selon l'une des revendications 1 à 4, **caractérisée en ce qu'**on utilise du mannitol comme excipient hydrosoluble.

6. Préparation pharmaceutique selon l'une des revendications 1 à 5, **caractérisée en ce que** l'excipient est traité avec une quantité d'EDTA-Na correspondant essentiellement à la teneur en Ca⁺⁺ de l'excipient et le cas échéant avec de l'acide citrique.

7. Préparation pharmaceutique selon l'une des revendications 1 à 6, **caractérisée en ce que** la préparation se présente sous forme de comprimés et est confectionnée avec des auxiliaires non hygroscopiques et/ou dans un emballage hermétique ou difficilement perméable à la vapeur d'eau.

8. Procédé de production d'une préparation pharmaceutique selon l'une des revendications 1 à 7, **caractérisé en ce qu'**on dépose ou pulvérise sur un excipient dérivé du mannitol une solution alcoolique du principe actif, sur quoi le solvant alcoolique est vaporisé jusqu'à obtenir un facteur d'eau inférieur à 0,4, notamment inférieur à 0,3 et supérieur à 0,1 et ensuite transformé en comprimés.

9. Procédé selon la revendication 8, **caractérisé en ce qu'**avant la vaporisation du solvant, on applique de l'eau ou une solution aqueuse contenant de l'EDTA-Na et/ou de l'acide citrique.

10. Procédé selon la revendication 8 ou 9, **caractérisé en ce qu'**on emploie comme excipient du mannitol directement transformable en comprimés, notamment du pearlitol.

11. Procédé selon la revendication 8, 9 ou 10, **caractérisé en ce qu'**on utilise comme auxiliaire de transformation en comprimés, notamment comme lubrifiant, des auxiliaires hydrophobes, par exemple le stéarate de magnésium.

12. Procédé selon l'une des revendications 8 à 11, **caractérisé en ce qu'**on mélange à l'excipient, avant l'application ou la pulvérisation de la solution de principe actif, de l'EDTA-Na et le cas échéant de l'acide citrique dans une quantité suffisante pour complexer les ions divalents de l'excipient et d'autres sources, comme des solvants.

13. Procédé selon l'une des revendications 8 à 12, **caractérisé en ce que** l'excipient est humecté avec du méthanol avant d'être mélangé à de l'EDTA-Na et le cas échéant à de l'acide citrique.

14. Procédé selon l'une des revendications 8 à 13, **caractérisé en ce que** la préparation est conservée sans adjuvants hygroscopiques.

15. Procédé selon l'une des revendications 8 à 13, **caractérisé en ce que** la préparation contient des adjuvants hygroscopiques et est conditionnée dans un emballage hermétique à la vapeur d'eau.
